# EUROPEAN PATENT SPECIFICATION

(11) **EP 3 341 040 B1**
(45) Date of publication and mention of the grant of the patent: **21.07.2021**
(21) Application number: 16763415.3
(22) Date of filing: 29.08.2016
(51) Int. Cl.: A61M 1/06

(54) **LACTEAL EXTRACTOR SAFETY SYSTEM**
SICHERHEITSSYSTEM FÜR MILCHEXTRAKTOR
SYSTÈME DE SÉCURITÉ DE TIRE-LAIT

(30) Priority: 27.08.2015 US 201562210841 P
(43) Date of publication of application: 04.07.2018
(73) Proprietor: Medela Holding AG, 6340 Baar (CH)
(72) Inventor: HOLTZ, Raymond, Marion, MA 02738 (US)
(74) Representative: Clerc, Natalia
(86) International application number: PCT/US2016/049183
(87) International publication number: WO 2017/035520

(56) References cited:
- WO-A1-00/66195
- WO-A1-2015/022244
- WO-A2-2014/094186
- CN-A- 104 784 766
- GB-A- 2 155 792
- US-A1- 2007 179 439
- US-A1- 2010 324 478
- US-A1- 2014 263 611
- US-A1- 2014 276 629
- US-A1- 2015 219 230

## Description

### CROSS-REFERENCE TO RELATED APPLICATION

This application claims priority to U.S. Provisional Patent Application No. 62/210,84 filed August 27, 2015 and entitled "Lacteal Extractor Safety System and Method for Pump System."

### TECHNICAL FIELD

The present disclosure relates generally to a mechanical safety system and method for a pump system. WO2015/022244 A1 discloses a breast pump device with a safety valve. More particularly, the disclosure relates to a mechanical over-vacuum safety system and method for a vacuum pump system designed to extract milk from lactating mothers.

### BRIEF DESCRIPTION OF THE DRAWINGS

FIG. 1 is a side view of an exemplary embodiment constructed in accordance with the principles herein;
FIG. 1A is an side view of an exemplary mechanical safety valve system constructed in accordance with the principles herein;
FIG. 2 is a side view, partially in section, of another exemplary embodiment constructed in accordance with the principles herein;
FIGS. 3a-b are sectional views taken along lines 3A of Figure 2 with the valve in a unsealed condition and sealed condition, respectively;
FIG. 3c is an exemplary embodiment of a portion of a fluid flow member configuration that includes at least one component that prevents the valve from fully entering the flow unit given an over vacuum condition in a pump system;
FIGS. 3d and 3e are pictures of a valve sealing the flow unit of the system under vacuum, and automatically venting under an over vacuum condition of the system, respectively.
FIG. 4 is a flow chart of an exemplary method of constructing a lacteal extractor safety system in accordance with the principles herein.

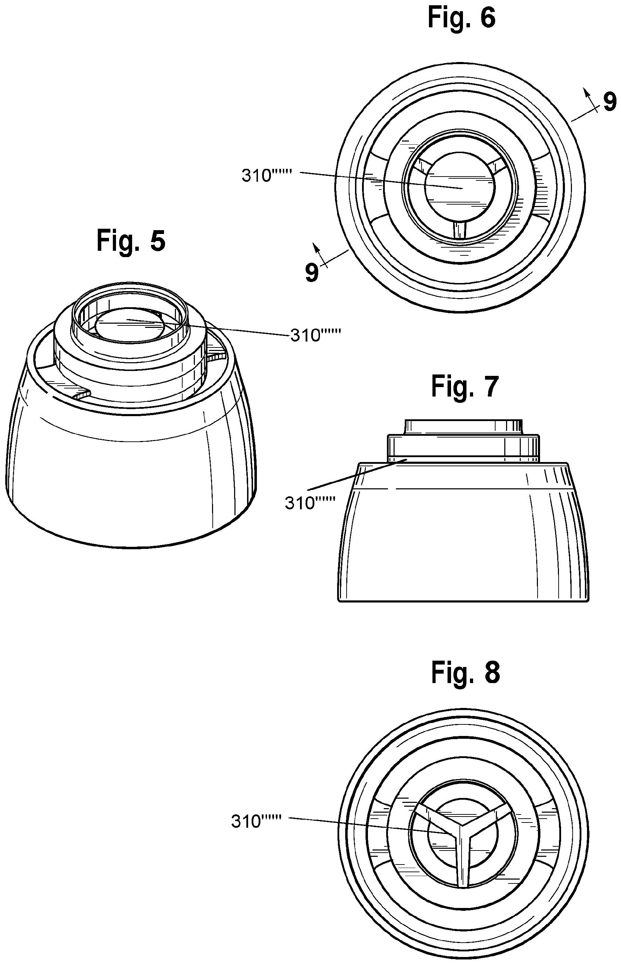

FIG. 5 is a front perspective view of an embodiment of a valve sealing the flow unit of the system under a vacuum and automatically venting under an over vacuum condition of the system.
FIG. 6 is atop view of the valve illustrated in FIG. 5.
FIG. 7 is a side view of the valve illustrated in FIGS. 5 and 6.
FIG. 8 is a bottom view of the valve illustrated in FIGS. 5-7.

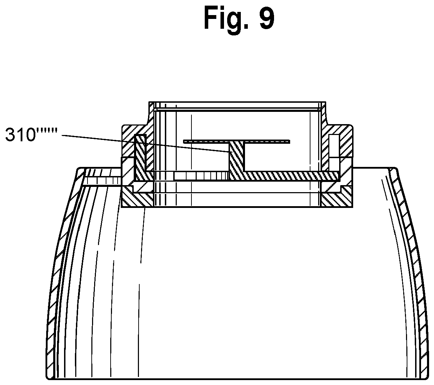

FIG. 9 is a cross-sectional view of the valve illustrated in FIGS. 5-8 taken along line 9-9 in FIG. 6.

Throughout the various figures, like reference numbers refer to like elements of the exemplary system components. Elements can be combined in all possible combinations that can be formed to achieve exemplary systems not specifically set forth in the figures, in accordance with the principles herein. The invention, however, is defined in claim 1. Preferred embodiments of the invention are specified in the dependent claims.

### DETAILED DESCRIPTION

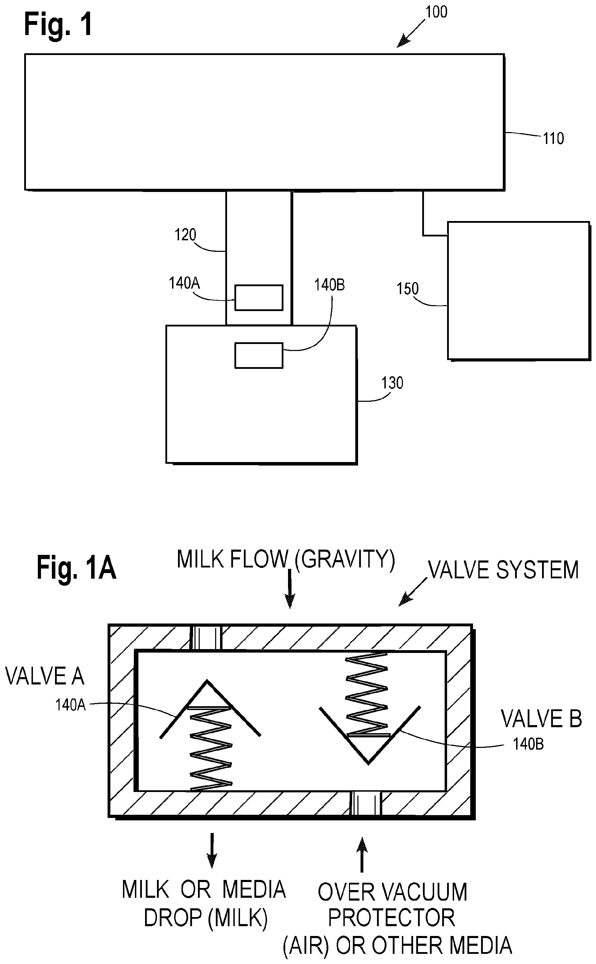

FIG. 1 illustrates an exemplary vacuum pump system shown generally at 100, constructed in accordance with the principles herein. The pump system 100 includes a lacteal extractor 110 connectable to a lactating mother, and a fluid flow member 120 through which milk extracted from the mother can flow. The fluid flow member 120 can be formed of any length, shape, and material, and can provide or contain one or more components designed to convey milk from the lacteal extractor 110 of the pump system 100 to a collection container 130. A vacuum generating system 150 can be either directly or indirectly connected to the lacteal extractor 110.

In accordance with the principles herein, vacuum generated by the vacuum generating system 150 and delivered to the lacteal extractor 110 can advantageously be automatically mechanically regulated to reduce the chance of an over vacuum condition in the system 100. To this end, one or more mechanical valves can be positioned between the milk collection container 130 and the lacteal extractor 110, where the one or more mechanical valves are designed to provide a mechanical safety valve system that is automatically actuated in response to specific vacuum ranges that can cause an over vacuum condition in the system. Although numerous pump systems employ mechanical valves to facilitate milk flow from a lacteal extractor to a container, the flow valves of conventional systems do not function as mechanical safety valves within the fluid flow path, and are not configured and tuned to mechanically open when subjected to an over vacuum condition of the system.

Alternately, in exemplary instances not forming part of the invention, the mechanical safety valve system could be positioned outside of the fluid flow path of the system, such as, for example, in the vacuum line or anywhere else in the system. In certain circumstances, the mechanical safety valve system can be positioned to serve as both a safety valve and a milk extraction valve, as illustrated according to the exemplary embodiments herein.

In an exemplary embodiment constructed in accordance with the principles herein, a safety valve 140A can be positioned within the flow unit 120 of the system 100. In yet another exemplary embodiment, a safety valve 140 B can be positioned within the collection container 130 of the system 100. In still another exemplary embodiment depicted in FIG. 1A, a mechanical safety valve system of more than one safety valve, such as a safety valve 140A and a safety valve 140B, or any number of valves that form a suitable safety valve system, can be position in the system 100 downstream from the lacteal extractor 110 to form the mechanical safety valve system. Suitable mechanical safety valve systems constructed in accordance with the principles herein automatically and mechanically reduce vacuum in the system by altering its geometry to allow communication with atmospheric pressure under certain vacuum ranges.

Typical vacuum ranges in which an over vacuum condition can occur in the system can include, for example, from around negative 300 mmHg to about negative 350mmHg. If desired, the vacuum ranges in which an over vacuum condition can occur can be fine-tuned with feedback from a mother using the system based her comfort levels. To this end, an adjustment, mechanical, electrical or both, of vacuum ranges can be made in the system based on the feedback from the mom. Such adjustment will not affect the efficacy of the valve, but can serve to reduce the pressure at the upper limit of the over vacuum condition, rendering operation of the device more comfortable for the mom.

In order for the mechanical safety valve system to function automatically in the system one must employ the right combination of material thickness of the valve, material properties of the valve, open or flow through diameter of the valve, and the beam or bend length of surface of the valve that achieves the safety feature in relation to the applied pressure on the valve during operation.

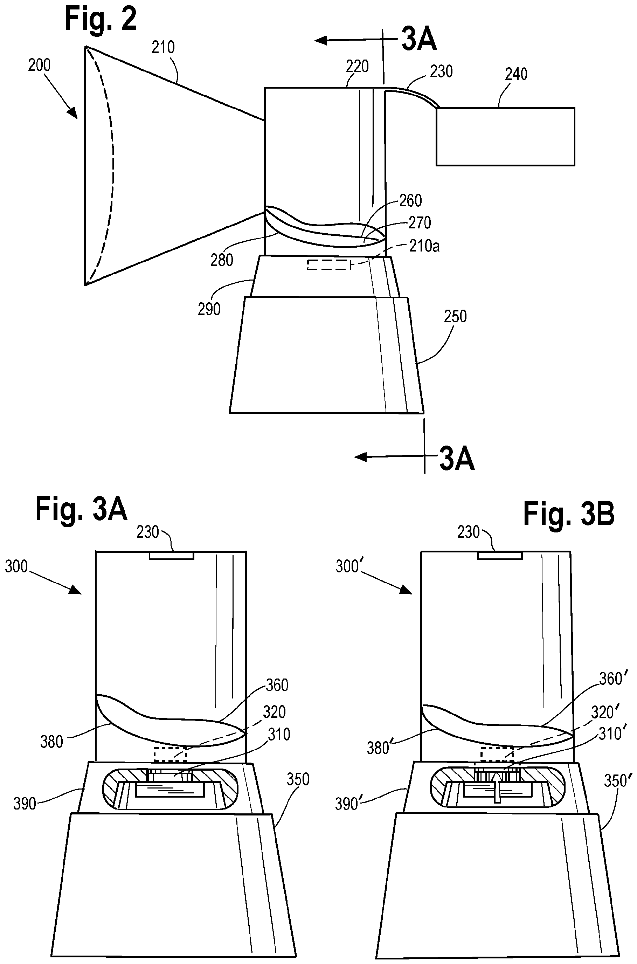

As illustrated in Figure 2, another exemplary embodiment of a system shown generally at 200 and constructed in accordance with the principles herein can include an exemplary lacteal extractor formed of a breastshield 210, an adapter 220 connected via tubing 230 to a vacuum pump 240 and a collection container 250. A separator unit 260 can be included to separate milk, or other fluid media, flowing from the breastshield 210 from entering the tubing 230 or the vacuum pump 240. An opening 270 can be provided along a bottom interior wall 280 of the adapter 220. The bottom interior wall 280 can be provided in the system 200 to minimize the work required by the vacuum generating system 240 to achieve desired vacuum ranges in accordance with a program or pattern of the pump system 200. The pump system 200 can be designed so that the program or pattern is selectable by a mother.

The system 200 can further include a valve system 290 containing a flexible, movable valve 210a. The valve system 290 is connectable to the adapter 220 and the container 230. Known lacteal extraction systems include a flow valve to the container. Some known systems include a relief or safety valve, but not an automatic mechanical safety valve within the system. For example, a relief valve can be connected with a breastshield as discussed in U.S. Patent No. 8,070,716 to Sutrina et al. Thus, in accordance with the principles herein the exemplary valve 210a can serve, advantageously, as both a fluid flow control valve and as an automatic mechanical safety valve system within the fluid flow.

As illustrated in Figures 3a to 3e, an exemplary mechanical safety valve system can include a flexible, movable valve 310. The valve 310 can control fluid flow in the system, and can allow fluid to pass into a collection container 350. The valve 310 can also serve as a mechanical safety valve system when the valve is constructed such that it limits the volume of the system in order to achieve upper vacuum levels, or negative pressure values, and alters the geometry of the valve to reduce vacuum levels when an over vacuum condition occurs in the system

In an exemplary embodiment shown in Figure 3b, a valve 310' can seal an aperture 320' leading to a container in the system when vacuum is applied. In this sense, the valve 310' can function similarly to conventional lacteal extraction flow control valves.

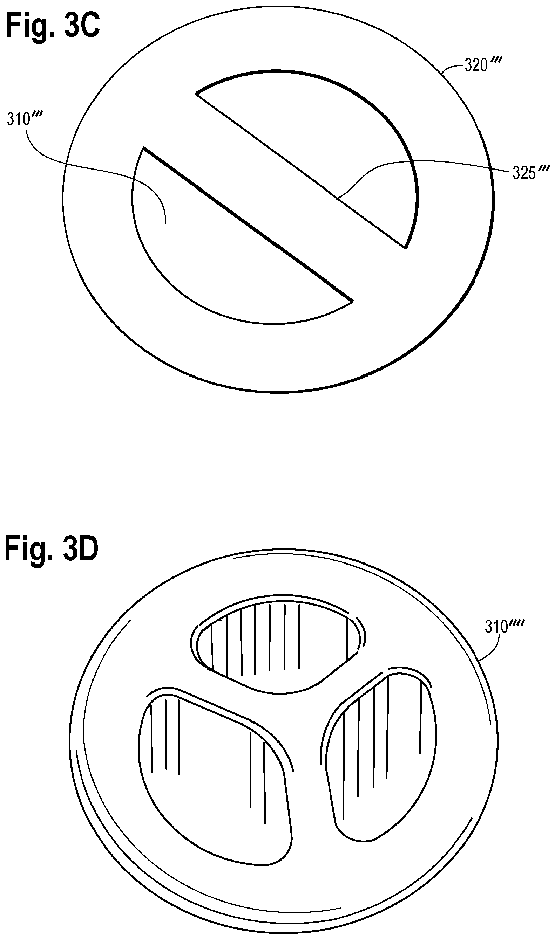

In the exemplary embodiment shown in Figure 3c, an aperture or opening 320' within a wall of a flow unit can include one or more ribs 325"', designed to prevent a valve 310'" from being pulled into the flow unit when an over vacuum condition occurs. In this embodiment the valve 310'" is sufficiently flexible under high vacuum conditions that the valve can flex and fold when sufficient vacuum is applied. As a result, ribs 325"' or other similar structure(s), such as a mesh or any other alternate structure(s), ensure that the valve remains in a position within the system to limit the system volume under normal vacuum conditions, and to alter the geometry of the valve in the system under over vacuum conditions.

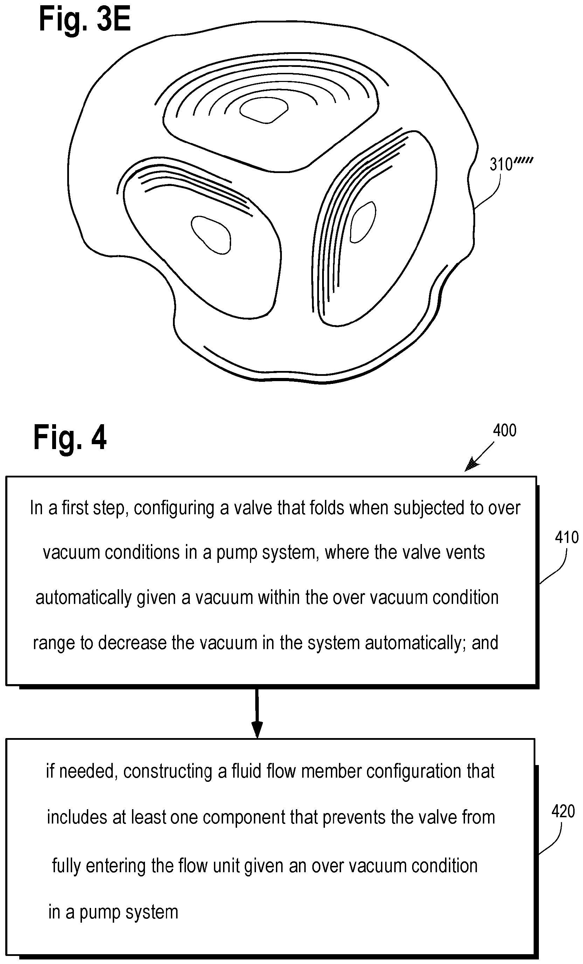

Moreover, in the embodiment of Figures 3d and 3e, the system is configured such that the valve 310"", 310'"" respectively can actually fold or bend as indicated to reduce vacuum of the system mechanically and automatically when an over vacuum condition occurs during operation. The valve can be designed to have physical characteristics and shape such that a portion of the valve can undergo stretching in an over vacuum condition that results in the bending or folding of the valve 310 as shown in Figure 3e. In the exemplary embodiments of Figures 3d and 3e, the aperture of the fluid unit includes three ribs that ensure the valve remains in a position within the system to limit the system volume under normal vacuum conditions, and to alter the geometry of the valve in the system under over vacuum conditions.

Although any suitable method can be employed in accordance with a method of the present disclosure, it is contemplated that particular advantage can be achieved through the use of manufacturing methods such as, for example an exemplary method of forming a mechanical safety valve system for a pump system illustrated in Figure 4. A method in accordance with the principles herein, shown generally at 400 can include the step at 410 of configuring a valve that folds when subjected to over vacuum conditions in a pump system, where the valve vents automatically given a vacuum within the over vacuum condition range to decrease the vacuum in the system automatically; and at step 420, if needed, constructing a fluid flow member configuration that includes at least one component that prevents the valve from fully entering the flow unit given an over vacuum condition in a pump system.

## Claims

1. A pump system (100, 200, 300, 300') for lacteal extraction comprising:
a mechanical safety valve (140A, 140B, 210a, 310, 310', 310'", 310"", 310"'", 310""") within a milk flow path of the system, wherein
the safety valve (140A, 140B, 210a, 310', 310'", 310"", 310'"", 310""") is configured to automatically actuate in response to specific vacuum ranges that can cause an over-vacuum condition,
the pump system (100, 200, 300, 300') further comprising
an adapter (220) connected by tubing (230) to a vacuum pump (240), the adapter further connected to a collection container (130, 250, 350, 350'), wherein the mechanical safety valve (140A, 140B, 210a, 310', 310'", 310"", 310'"", 310""") is in the adapter (220),
wherein the adapter (220) comprises an opening (270) on a bottom wall (280) of the adapter (220), and the mechanical safety valve (140A, 140B, 210a, 310', 310'", 310"", 310'"", 310""") is at the opening (270), and
wherein the mechanical safety valve (140A, 140B, 210a, 310', 310'", 310"", 310'"", 310""") allows fluid flow by folding.

2. The pump system (100, 200, 300, 300') of Claim 1, further comprising:
a lacteal extractor (110, 210) connected to the adapter;
wherein the mechanical safety valve (140A, 140B, 210a, 310', 310'", 310"", 310'"", 310""") is positioned between the lacteal extractor (110, 210) and the collection container (130, 250, 350, 350').

3. The pump system of Claims 1 or 2, wherein the pump system (100, 200, 300, 300') includes two or more mechanical safety valves (140A, 140B, 210a, 310', 310'", 310"", 310'"", 310""") located downstream of a lacteal extractor (110, 210).

4. The pump system (100, 200, 300, 300') of Claims 2 or 3, wherein the lacteal extractor (110, 210) is a breastshield (210).

5. The pump system (100, 200, 300, 300') of Claim 1, wherein the opening (270) is partially covered by ribs (325"'), a mesh, or an alternate structure.

6. The pump system (100, 200, 300, 300') of any of Claims 1-5 wherein the over-vacuum condition occurs between negative 300 mmHG and negative 350 mmHG.

7. The pump system (100, 200, 300, 300') of any of Claims 1-6, wherein the mechanical safety valve (140A, 140B, 210a, 310', 310'", 310"", 310'"", 310""") is flexible.

8. The pump system (100, 200, 300, 300') of any of Claims 1-7, wherein the mechanical safety valve (140A, 140B, 210a, 310', 310'", 310"", 310'"", 310""") undergoes stretching in an over-vacuum condition.

9. The pump system of Claim 5, wherein the ribs (325'"), the mesh, or the alternate structure prevent the valve from fully traveling through the opening (270).

10. The pump system of Claim 3, wherein one of the two or more mechanical valves (140B) is positioned within the collection container (130).

11. The pump system (110, 200, 300, 300') of Claim 10, where the pump system (100, 200, 300, 300') comprises a lacteal extractor (110) directly or indirectly connected to a vacuum generating system (150).

## Patentansprüche

1. Ein Pumpsystem (100, 200, 300, 300') zur Milchextraktion, aufweisend ein mechanisches Sicherheitsventil (140A, 140B, 210a, 310, 310', 310"', 310"", 310""', 310""") innerhalb eines Milchflusspfads des Systems, wobei das Sicherheitsventil (140A, 140B, 210a, 310, 310', 310"', 310"", 310'"", 310""") ausgebildet ist, sich automatisch in Reaktion auf spezifische Unterdruckbereiche, die ein Über-Vakuumzustand verursachen können, zu bewegen,
das Pumpsystem (100, 200, 300, 300') ferner aufweisend
einen Adapter (220), der über eine Leitung (230) mit einer Vakuumpumpe (240) verbunden ist, wobei der Adapter ferner mit einem Sammelbehälter (130, 250, 350, 350') verbunden ist, wobei das mechanische Sicherheitsventil (140A, 140B, 210a, 310, 310', 310'", 310"", 310""', 310""") im Adapter (220) ist,
wobei der Adapter (220) eine Öffnung (270) an einer unteren Wand (280) des Adapters (220) aufweist und das mechanische Sicherheitsventil (140A, 140B, 210a, 310, 310', 310'", 310"", 310""', 310""") an der Öffnung (270) ist, und
wobei das mechanische Sicherheitsventil (140A, 140B, 210a, 310, 310', 310'", 310"", 310""', 310""") durch Falten einen Fluidstrom erlaubt.

2. Das Pumpsystem (100, 200, 300, 300') nach Anspruch 1, ferner aufweisend:
einen mit dem Adapter verbundenen Milchextraktor (110, 210),
wobei das mechanische Sicherheitsventil (140A, 140B, 210a, 310, 310', 310"', 310"", 310""', 310""") zwischen dem Milchextraktor (110, 210) und dem Sammelbehälter (130, 250, 350, 350') angeordnet ist.

3. Das Pumpsystem (100, 200, 300, 300') nach einem der Ansprüche 1 oder 2, wobei das Pumpsystem (100, 200, 300, 300') zwei oder mehr mechanische Sicherheitsventile (140A, 140B, 210a, 310, 310', 310"', 310"", 310'"", 310""") aufweist, die stromabwärts eines Milchextraktors (110, 210) angeordnet sind.

4. Das Pumpsystem (100, 200, 300, 300') nach einem der Ansprüche 2 oder 3, wobei der Milchextraktor (110, 210) eine Brusthaube (210) ist.

5. Das Pumpsystem (100, 200, 300, 300') nach Anspruch 1, wobei die Öffnung (270) teilweise mit Rippen (325'"), einem Netz oder mit einer alternativen Struktur bedeckt ist.

6. Das Pumpsystem (100, 200, 300, 300') nach einem der Ansprüche 1 - 5, wobei der Über-Vakuumzustand zwischen negativem 300 mmHG und negativem 350 mmHG auftritt.

7. Das Pumpsystem (100, 200, 300, 300') nach einem der Ansprüche 1 - 6, wobei das mechanische Sicherheitsventil (140A, 140B, 210a, 310, 310', 310'", 310"", 310'"", 310""") flexibel ist.

8. Das Pumpsystem (100, 200, 300, 300') nach einem der Ansprüche 1 - 7, wobei das mechanische Sicherheitsventil (140A, 140B, 210a, 310, 310', 310'", 310"", 310'"", 310""") im Über-Vakuumzustand eine Dehnung erfährt.

9. Das Pumpsystem (100, 200, 300, 300') nach Anspruch 5, wobei die Rippen (325'"), das Netz, oder die alternative Struktur verhindern, dass sich das Ventil vollständig durch die Öffnung (270) bewegt.

10. Das Pumpsystem (100, 200, 300, 300') nach Anspruch 3, wobei eines der zwei oder mehr mechanische Ventile (140B) im Sammelbehälter (130) angeordnet ist.

11. Das Pumpsystem (100, 200, 300, 300') nach Anspruch 10, wobei das Pumpsystem (100, 200, 300, 300') einen Milchextraktor (110) aufweist, der direkt oder indirekt mit einem Vakuumerzeugungssystem (150) verbunden ist.

## Revendications

1. Système de pompe (100, 200, 300, 300') pour tirer du lait comprenant :
une soupape de sécurité mécanique (140A, 140B, 210a, 310, 310', 310'", 310"", 310'"", 310""") à l'intérieur d'un trajet d'écoulement de lait du système, dans lequel
la soupape de sécurité (140A, 140B, 210a, 310', 310'", 310"", 310'"", 310""") est configurée pour s'actionner automatiquement en réponse à des plages de vide spécifiques qui peuvent provoquer une condition de vide excessif,
le système de pompe (100, 200, 300, 300') comprenant en outre
un adaptateur (220) connecté par un conduit (230) à une pompe à vide (240), l'adaptateur étant en outre connecté à un récipient de collecte (130, 250, 350, 350'), dans lequel la soupape de sécurité mécanique (140A, 140B, 210a, 310', 310'", 310"", 310'"", 310""") est dans l'adaptateur (220),
dans lequel l'adaptateur (220) comprend une ouverture (270) sur une paroi inférieure (280) de l'adaptateur (220), et la soupape de sécurité mécanique (140A, 140B, 210a, 310', 310"', 310"", 310""', 310""") est au niveau de l'ouverture (270), et
dans lequel la soupape de sécurité mécanique (140A, 140B, 210a, 310', 310'", 310"", 310'"", 310""") permet un écoulement de fluide par pliage.

2. Système de pompe (100, 200, 300, 300') selon la revendication 1, comprenant en outre :
un tire-lait (110, 210) connecté à l'adaptateur ;
dans lequel la soupape de sécurité mécanique (140A, 140B, 210a, 310', 310'", 310"", 310'"", 310""") est positionnée entre le tire-lait (110, 210) et le récipient de collecte (130, 250, 350, 350').

3. Système de pompe selon la revendication 1 ou 2, le système de pompe (100, 200, 300, 300') comprenant deux soupapes de sécurité mécaniques ou plus (140A, 140B, 210a, 310', 310'", 310"", 310'"", 310""") situées en aval d'un tire-lait (110, 210).

4. Système de pompe (100, 200, 300, 300') selon la revendication 2 ou 3, dans lequel le tire-lait (110, 210) est une téterelle (210).

5. Système de pompe (100, 200, 300, 300') selon la revendication 1, dans lequel l'ouverture (270) est partiellement recouverte par des nervures (325'"), un maillage ou une structure alternative.

6. Système de pompe (100, 200, 300, 300') selon la revendication 1 à 5 dans lequel la condition de vide excessif se produit entre moins 300 mmHg et moins 350 mmHg.

7. Système de pompe (100, 200, 300, 300') selon l'une quelconque des revendications 1 à 6, dans lequel la soupape de sécurité mécanique (140A, 140B, 210a, 310', 310'", 310"", 310'"", 310""") est flexible.

8. Système de pompe (100, 200, 300, 300') selon l'une quelconque des revendications 1 à 7, dans lequel la soupape de sécurité mécanique (140A, 140B, 210a, 310', 310'", 310"", 310'"", 310""") subit un étirement dans une condition de vide excessif.

9. Système de pompe selon la revendication 5, dans lequel les nervures (325"'), le maillage ou la structure alternative évitent que la soupape se déplace entièrement à travers l'ouverture (270).

10. Système de pompe selon la revendication 3, dans lequel une des deux soupapes mécaniques ou plus (140B) est positionnée à l'intérieur du récipient de collecte (130).

11. Système de pompe (110, 200, 300, 300') selon la revendication 10, le système de pompe (100, 200, 300, 300') comprenant un tire-lait (110) connecté directement ou indirectement à un système de production de vide (150).
